# FASCICULE DE BREVET EUROPEEN

(11) **EP 1 858 549 B1**
(45) Date de publication et mention de la délivrance du brevet: **02.10.2013**
(21) Numéro de dépôt: 06743593.3
(22) Date de dépôt: 17.03.2006
(51) Int. Cl.: A61K 39/008, C07K 14/44

(54) **COMPOSITION COMPRENANT LA REGION N-TERMINALE D'HISTONE H2B DE LEISHMANIA - UTILISATION POUR INDUIRE UNE REPONSE IMMUNITAIRE**
ZUSAMMENSETZUNG MIT DER N-THERMALEN REGION DES LEISHMANIA-HISTONS H2B UND IHRE VERWENDUNG ZUR HERBEIFÜHRUNG EINER IMMUNANTWORT
COMPOSITION COMPRISING THE N-TERMINAL REGION OF LEISHMANIA HISTONE H2B, USE THEREOF FOR INDUCING AN IMMUNE RESPONSE

(30) Priorité: 18.03.2005 FR 0502725
(43) Date de publication de la demande: 28.11.2007
(73) Titulaire: INSTITUT PASTEUR, 75724 Paris Cedex 15 (FR); INSTITUT PASTEUR DE TUNIS, 1002 Tunis Belvedere (TN)
(72) Inventeur: CHENIK, Mehdi, 2070 La Marsa (TN); LOUZIR, Hechmi, 2070 La Marsa (TN); DELLAGI, Koussay, Tunis-Babsaadoun (TN)
(74) Mandataire: Paris, Fabienne
(86) Numéro de dépôt international: PCT/FR2006/000597
(87) Numéro de publication internationale: WO 2006/097642

(56) Documents cités:
- WO-A-2005/044301
- IBORRA S ET AL: "Vaccination with a plasmid DNA cocktail encoding the nucleosomal histones of Leishmania confers protection against murine cutaneous leishmaniosis" VACCINE, BUTTERWORTH SCIENTIFIC. GUILDFORD, GB, vol. 22, no. 29-30, 28 septembre 2004 (2004-09-28), pages 3865-3876, XP004567462 ISSN: 0264-410X cité dans la demande
- SOTO M ET AL: "ANTIGENICITY OF THE LEISHMANIA INFANTUM HISTONES H2B AND H4 DURING CANINE VISCEROCUTANEOUS LEISHMANIASIS" CLINICAL AND EXPERIMENTAL IMMUNOLOGY, OXFORD, GB, vol. 2, no. 115, février 1999 (1999-02), pages 342-349, XP001086535 ISSN: 0009-9104 cité dans la demande
- MAALEJ IBTISSEM ABDMOULEH ET AL: "Comparative evaluation of ELISAs based on ten recombinant or purified Leishmania antigens for the serodiagnosis of Mediterranean visceral leishmaniasis." AMERICAN JOURNAL OF TROPICAL MEDICINE AND HYGIENE, vol. 68, no. 3, mars 2003 (2003-03), pages 312-320, XP002365318 ISSN: 0002-9637 cité dans la demande
- DATABASE EMBL [Online] 21 mars 2001 (2001-03-21), "Leishmania major histone H2B (H2B) mRNA, complete cds." XP002384653 extrait de EBI accession no. EM_PRO:AF336276 Database accession no. AF336276
- PROBST P ET AL: "Identification and characterization of T cell-stimulating antigens from Leishmania by CD4 T cell expression cloning." THE JOURNAL OF IMMUNOLOGY, vol. 166, no. 1, 1 janvier 2001 (2001-01-01), pages 498-505, XP002365319 ISSN: 0022-1767

### SEQUENCE LISTING

<110> INSTITUT PASTEUR DE PARIS INSTITUT PASTEUR DE TUNIS
<120> COMPOSITION COMPRENANT LA REGION N-TERMINALE D'HISTONE H2B DE LEISHMANIA - UTILISATION POUR INDUIRE UNE REPONSE IMMUNITAIRE
<130> B6259A-AD/YH/KP
<140> New Pct patent application
   <141> 2006-03-17
<150> FR2005/02725
   <151> 2005-03-18
<160> 2
<170> PatentIn version 3.3
<210> 1
   <211> 111
   <212> PRT
   <213> Naturel
<400> 1
   Met Ala Ser Ser Arg Ser Ala Ser Arg Lys Ala Ser Asn Pro His Lys
   1               5                   10                  15
   Ser His Arg Lys Pro Lys Arg Ser Trp Asn Val Tyr Val Gly Arg Ser
               20                  25                  30
   Leu Lys Ala Ile Asn Ala Gln Met Ser Met Ser His Arg Thr Met Lys
           35                  40                  45
   Ile Val Asn Ser Tyr Val Asn Asp Val Met Glu Arg Ile Cys Thr Glu
       50                  55                  60
   Ala Ala Ser Ile Val Arg Ala Asn Lys Lys Arg Thr Leu Gly Ala Arg
   65                  70                  75                  80
   Glu Val Gln Thr Ala Val Arg Ile Val Leu Pro Ala Glu Leu Ala Lys
                   85                  90                  95
   His Ala Met Ala Glu Gly Thr Lys Ala Val Ser Ser Ala Ser Ala
               100                 105                 110
<210> 2
   <211> 336
   <212> DNA
   <213> Naturel
<400> 2
   atggcctctt ctcgctctgc ttcccgcaag gcttccaacc cgcacaagtc gcaccgcaag      60
   ccgaagcgct cgtggaacgt gtacgtgggc cgctcgctga aggcgatcaa cgcccagatg     120
   tcgatgtcgc accgcacgat gaagatcgtg aactcgtacg tgaacgacgt gatggagcgc     180
   atctgcaccg aggccgcgtc gattgttcgc gcgaacaaga agcgcacgtt gggtgcgcgc     240
   gaggtgcaga cggcggtgcg cattgtgctg ccggcggagc tcgcgaagca cgccatggct     300
   gagggcacga aggccgtgtc gagcgcgtcg gcttga                               336

## Description

La demande décrit une composition immunogène comprenant : i) un polypeptide antigénique consistant en un fragment de la protéine histone H2B de *Leishmania,* ledit fragment comprenant tout ou partie de la région N-terminale de ladite protéine histone H2B, et ii) un adjuvant stimulant la réponse immunitaire.

La demande décrit également un polypeptide consistant en un fragment de la protéine histone H2B de *Leishmania,* ledit fragment comprenant tout ou partie de la région N-terminale de ladite protéine histone H2B, un polypeptide variant conservant au moins 50% d'identité, de préférence au moins 80% d'identité avec ledit polypeptide, et une molécule d'acide nucléique codant le polypeptide ou le polypeptide variant.

La demande décrit aussi un vaccin permettant d'obtenir une protection contre une infection par *Leishmania,* dont le principe actif comprend le polypeptide, le polypeptide variant ou la molécule d'acide nucléique selon la présente demande, et un vecteur d'expression capable d'exprimer le polypeptide ou le polypeptide variant, comprenant la molécule d'acide nucléique.

Les leishmanioses constituent un groupe hétérogène de pathologies qui affectent plusieurs millions d'individus et sont dues à l'infection de l'hôte animal ou humain par un parasite protozoaire du genre *Leishmania* (Desjeux et coll, 1996). L'expression clinique de l'infection est caractérisée par un grand polymorphisme incluant l'infection asymptomatique, les formes cutanées simples ou récidivantes, les formes cutanées diffuses ou anergiques, les formes cutanéo-muqueuses et les formes viscérales mortelles en l'absence de traitement spécifique (WHO Expert Committee, 1990). Durant son cycle, le parasite alterne entre deux stades : le stade promastigote flagellé qui se trouve dans le tube digestif de l'insecte vecteur et le stade amastigote dans le macrophage de l'hôte mammifère.

La mise au point d'un vaccin anti-leishmanien est envisageable car il a été clairement démontré chez l'homme, que l'infection par *Leishmania* confère une immunité protectrice à la réinfection (Guirges et coll, 1971 ; Davies et coll, 1995). Cette protection est associée au développement d'une réponse immune cellulaire spécifique du parasite.

Plusieurs candidats vaccins ont été évalués contre l'infection par le parasite *Leishmania,* aussi bien dans le modèle murin que chez l'homme.

A l'heure actuelle, aucun vaccin à usage humain ou vétérinaire n'a montré une efficacité significative dans la protection contre les leishmanioses (Sharifi et coll, 1998 ; Khalil et coll, 2000; De Luca et coll, 2001; Armijos et coll, 2004).

Ces vaccins peuvent être distingués en trois groupes : les vaccins de première génération sont constitués de promastigotes tués par la chaleur ou par irradiation ; malgré leur innocuité, l'efficacité de ces vaccins n'a pas encore été prouvée (Sharifi et coll, 1998 ; Khalil et coll, 2000; De Luca et coll, 2001; Armijos et coll, 2004).

Les vaccins de deuxième génération, sont constitués de protéines natives obtenues par purification biochimique, de protéines recombinantes et de peptides synthétiques obtenus par génie génétique. Plusieurs protéines ont été décrites dans la littérature comme induisant une protection significative dans le modèle expérimental murin. Les plus importantes sont représentées par la polyprotéine Leish-111f composée de 3 protéines fortement immunogènes placées en tandem, le LeIF *(Leishmania* elongation initiation factor), la protéine LmSTI1(*L*. *major* stress-inducible protein) et la protéine TSA (thiol-specific antioxidant). Plusieurs travaux, ont permis de montrer que chacune de ces protéines testées individuellement ou associée à l'IL-12 est capable d'induire un ralentissement très significatif de l'évolution de la maladie chez les souris BALB/c infectées par *L. major* (Skeiky et coll, 1998; Coler et coll, 2002; Skeiky et coll, 2002; Campos-Neto et coll, 2002). La protéine LACK (leishmanial homolog of receptor for activated c kinase) en présence de l'IL-12 est également capable de conférer une protection des souris BALB/c contre un challenge virulent par *L. major* (Gurunathan et coll, 1997). Enfin, l'antigène PSA-2 purifié à partir de *Leishmania* confère aux souris une bonne protection (Handman, 1995).

Les vaccins de troisième génération comprennent les vaccins à base d'ADN contenant un ou plusieurs gènes codant pour des antigènes leishmaniens (Iborra et coll, 2003; Campbell et coll, 2003; Ramiro et coll, 2003 ; Dumonteil et coll, 2003).

Les vaccins de troisième génération comprennent également les leishmanies dont le pouvoir pathogène a été atténué génétiquement par la délétion de gènes de virulence. On citera le cas des leishmanies délétées du gène codant pour la dihydrofolate reductase thymidilate synthétase (dhfr-ts⁻). Plusieurs études ont montré dans le modèle murin que la vaccination des souris par ces parasites dhfr-ts⁻ pouvait induire une protection partielle contre l'infection par *Leishmania* (Brodskyn et coll, 2000; Streit et coll, 2001). Il est également possible d'inclure dans les vaccins de troisième génération, les vaccins à base de peptides synthétiques. Plusieurs essais de vaccination ont été réalisés avec différents peptides incluant généralement des épitopes T. L'exemple le plus connu est celui du peptide PT3 qui correspond à « l'histidine zinc binding région » de la gp63. Ainsi, ce peptide en présence d'un adjuvant « poloxamer 407 » est capable de conférer une protection durable des souris sensibles BALB/c, suite à un challenge par *L*. *major* (Spitzer et al, 1999).

Certains auteurs se sont intéressés aux protéines histones : le premier travail remonte à 1999, dans lequel Soto et coll (1999) ont montré que la protéine H2B pouvait constituer une cible de la réponse anticorps au cours de la leishmaniose canine. Plus tard Maalej et coll ont évalué la protéine H2B, en comparaison avec d'autres protéines parasitaires, la proposant comme antigène pour le diagnostic sérologique de la leishmaniose viscérale humaine et ont montré qu'elle pourrait constituer une cible intéressante (Maalej et coll, 2003).

Concernant la réponse cellulaire, Probst et coll (2001) ont montré que la protéine H2B, sous sa forme recombinante, était capable d'induire une forte prolifération de cellules mononuclées de sang périphérique (PBMC) chez des patients ayant guéri d'une leishmaniose cutanée causée par *L*. *major.* Cette prolifération était accompagnée d'une importante production IFN-γ avec absence d'IL-4 (Probst et coll 2001).

Dans le modèle murin de la leishmaniose viscérale (LV) causée par *L. donovani,* Melby et coll (2000), ont montré que la vaccination de souris sensibles BALB/c, par une fraction de DNA vaccins obtenus à partir d'une banque cDNA, contenant notamment H2B, était capable de protéger partiellement ces souris après un challenge virulent par *L. donovani.*

Très récemment, dans le modèle de la leishmaniose expérimentale d'infection des souris BALB/c par *L .major,* Iborra et coll, (2004) ont montré que la vaccination des souris par un cocktail d'ADN vaccin codant pour les protéines histones (H2A, H2B, H3 et H4) induisait une importante réponse immune de type Th1 avec sécrétion d'INFγ associée à une protection partielle des souris vaccinées suite à un challenge par *L. major.*

Par ailleurs, il a été démontré que le parasite *Leishmania* est capable d'induire l'activation des cellules T régulatrices (Belkaid et coll, 2002), qui vont supprimer les réponses immunes effectrices et permettre la multiplication et la persistance du parasite.

Des données concordantes suggèrent que les cellules T régulatrices sont sélectionnées positivement après une forte interaction avec les peptides du soi (TCR-CMH-peptide) (Gavin et Rudensky, 2003).

Les inventeurs se sont intéressés à la protéine histone H2B comme candidat vaccin. Plus précisément, ils ont tout d'abord émis l'hypothèse que certaines régions conservées des protéines parasitaires pourraient activer préférentiellement les cellules régulatrices alors que les séquences divergentes activeraient les cellules effectrices, et également, qu'une même protéine parasitaire pourrait contenir des peptides inducteurs de réponses régulatrices et d'autres inducteurs de réponses effectrices.

Les inventeurs ont analysé comparativement l'immunogénicité et le rôle protecteur des deux régions amino-terminale, divergente, et carboxy-terminale, conservée, de la protéine recombinante H2B de *L.major* ainsi que de la protéine entière.

Ils se sont aperçus que, de façon surprenante, la partie amino-terminale (divergente) de H2B, adjuvée au CpG, confère la meilleure protection des souris sensibles BALB/c contre un challenge virulent par *L. major.* Cette protéine tronquée pourrait constituer un bon candidat vaccin contre les leishmanioses.

La demande décrit une composition immunogène caractérisée en ce qu'elle comprend : i) un polypeptide antigénique consistant en un fragment de la protéine histone H2B de *Leishmania,* ledit fragment comprenant tout ou partie de la région N-terminale de ladite protéine histone H2B, et ii) un adjuvant stimulant la réponse immunitaire.

Dans un mode de réalisation particulier de la demande, le principe actif immunogène de ladite composition immunogène consiste en ledit polypeptide antigénique consistant en un fragment dérivé de la seule partie N-terminale de la protéine histone H2B de *Leishmania.*

Le terme « fragment » appliqué à un polypeptide ou plus particulièrement à un antigène, vise une partie de la protéine native considérée, excluant la totalité de ladite protéine.

La partie N-terminale d'un polypeptide natif, par exemple de la protéine histone H2B de *Leishmania* représente de façon générale moins de la moitié de la séquence d'acides aminés totale de ce polypeptide, la séquence retenue étant celle de l'extrémité N-terminale.

Dans un exemple spécifique, le polypeptide selon la demande a une séquence polypeptidique de moins de 65 acides aminés, de préférence moins de 50 acides aminés. Le polypeptide a dans ce cadre une séquence comprenant au moins 6 acides aminés consécutifs de la partie N-terminale de l'antigène H2B considéré.

Dans un exemple spécifique, la longueur de la séquence dudit polypeptide représente 10 à 45% de la longueur de la protéine histone.

La séquence d'acides aminés d'un polypeptide selon la demande est par exemple la suivante : M A S S R S A S R K A S N P H K S H R K P K R S W N V Y V G R S L K A I N A Q M S M S H R T.

Dans un exemple spécifique, le polypeptide est caractéristique de l'histone H2B de *Leishmania major.*

Le polypeptide peut être également caractéristique de la protéine histone H2B de *L. Infantum, L. Donovani, L. Tropica, L. Enrietti.*

Les séquences des protéines histone H2B de ces parasites ont été décrites: H2B de *L. Infantum* a été décrite dans Soto M. et al, 1999 ; Antigenicity of the Leishmania infantum histones H2B and H4 during canine viscerocutaneous leishmaniasis ; Journal Clin. Exp. Immunol. 115(2), 342-349 (1999) ; H2B *L. Enrietti* a été décrite dans Genske J. E. et al ; 1991 ; Structure and regulation of histone H2B mRNAs from Leishmania enrietti ; Mol. Cell. Biol. 11(1), 240-249 (1991).

Les séquences protéiques H2B de *L. Donovani* et *L. Tropica* n'ont pas encore été décrites. Cependant, la conservation attendue au niveau des protéines histones H2B de *Leishmania* devrait être supérieure à 80%.

De manière générale, le polypeptide peut être caractéristique d'une espèce choisie dans l'ensemble du genre *Leishmania.*

Dans un exemple spécifique, le polypeptide compris dans la composition immunogène selon la demande est un polypeptide purifié ou isolé de son environnement de production.

Dans un exemple spécifique, ladite composition immunogène comprend un ADN sous forme d'une séquence polynucléotidique codant pour le polypeptide selon la demande. Par exemple, le polynucléotide est inséré dans un plasmide d'expression eucaryote.

Un ou plusieurs acides aminés du polypeptide déterminé à partir d'une protéine histone H2B d'une espèce de *Leishmania,* tel qu'illustré ci-dessus et désigné par l'expression "polypeptide de référence", peuvent être substitués, délétés ou ajoutés pour former un polypeptide variant conservant au moins 50% d'identité, de préférence au moins 80% d'identité avec le polypeptide de référence, ledit polypeptide variant conservant les propriétés immunogènes du polypeptide de référence.

Le pourcentage d'identité entre le polypeptide de référence et le polypeptide variant est déterminé après avoir réalisé un alignement global optimal. Cet alignement peut être notamment obtenu en utilisant l'algorythme « Needleman-Wunsch » (Needleman and Wunsch, 1970, J. Mol. Biol. 48:443-453) ou ClustalW (Higgins & Sharp, a package for performing multiple sequence alignment on a microcomputer, 1988, Dec 15;73(1):237-44).

La notion d'identité signifie qu'un acide aminé à une position déterminée dans le polypeptide de référence est identique à un acide aminé correspondant dans le polypeptide variant ayant fait l'objet d'un alignement par rapport au polypeptide de référence.

Le fait que la séquence du polypeptide de référence soit n% identique à la séquence du polypeptide variant signifie que n% des positions de l'alignement global optimal entre les deux séquences consistent en des acides aminés identiques.

L'expression « propriétés immunogènes d'un polypeptide » signifie que le polypeptide est capable de provoquer, seul ou en association avec un adjuvant de l'immunité ou une molécule porteuse si besoin, une réaction immunitaire, notamment une réponse immunitaire de type cellulaire, par exemple une réponse des cellules T CD4+.

Par exemple, le polypeptide variant a une séquence polypeptidique de moins de 75 acides aminés, de préférence moins de 60 acides aminés.

A titre illustratif, les polypeptides variants peuvent comporter des substitutions conservatives d'acides aminés dans la séquence de l'histone H2B de *Leishmania* illustrée ci-dessus.

Un ou plusieurs acides aminés du polypeptide de référence, peuvent être substitués, délétés ou ajoutés pour former un polypeptide variant conservant au moins 50% de similarité, de préférence au moins 80% de similarité avec le polypeptide de référence, ledit polypeptide variant conservant les propriétés immunogènes du polypeptide de référence.

Par opposition à la notion d'identité, la notion de similarité concerne des acides aminés identiques, et des acides aminés différents mais donnant lieu à un polypeptide qui conserve les propriétés immunogènes du polypeptide de référence ; ou seulement de tels acides aminés différents.

Le fait que la séquence du polypeptide de référence soit n% similaire à la séquence du polypeptide variant signifie que n% des positions de l'alignement global optimal entre les deux séquences consistent en des acides aminés identiques, et des acides aminés différents dont la présence dans le polypeptide ne modifie pas sensiblement ses propriétés immunogènes ; ou seulement de tels acides aminés différents.

En particulier, les substitutions dans le polypeptide sont des substitutions conservatives.

Une substitution conservative est toute substitution ayant un score positif dans la matrice de substitution Blosum62, « blosum62 substitution matrix »,

(Henikoff and Henikoff, 1992, Proc. Natl. Acad. Sci. USA 89: 10915-10919).

Un autre mode de réalisation des variants polypeptidiques fait appel à la préparation de peptides de la protéine histone H2B considérée, lesdits peptides ayant par exemple des séquences peptidiques comportant de 6 à 30, par exemple de 6 à 20 ou de 6 à 15 acides aminés, choisies à partir de la séquence N-terminale de la protéine histone.

Les peptides peuvent être utilisés en mélanges, y compris sous la forme de mixotopes. Les mélanges peptidiques comprennent par exemple au moins 2 ou au moins 5 séquences peptidiques différentes les unes des autres ou au contraire ayant des acides aminés consécutifs en commun.

Les polypeptides (y compris les peptides) de l'invention sont préparés par toutes méthodes connues de l'homme du métier, par exemple par synthèse chimique, ou expression dans un système cellulaire, notamment bactérien, ou *in vitro* dans un système "cell free".

Le terme « adjuvant » signifie toute substance ou composé capable de promouvoir ou d'augmenter la réponse immunitaire contre un principe actif déterminé dans un mammifère, par comparaison avec la réponse immunitaire obtenue dans les mêmes conditions en administrant le principe actif seul.

Dans un exemple spécifique de réalisation de la demande, l'adjuvant favorise une réponse immunitaire de type cellulaire. L'adjuvant est par exemple le CpG ou tout autre adjuvant autorisé dans un usage vaccinal chez l'homme ou l'animal. La concentration du CpG utilisée pour la vaccination des souris est de 1 µg/µl et la quantité injectée est de 50 µg.

Dans un exemple spécifique, la composition immunogène selon la demande comprend en outre au moins un autre antigène de *Leishmania* ayant des propriétés immunogènes pour déclencher et/ou favoriser une réponse immunitaire, de type B et/ou T chez un hôte humain ou animal.

La composition immunogène selon la demande peut comprendre en outre au moins un polypeptide consistant en un fragment d'au moins une protéine histone supplémentaire de *Leishmania.*

Ledit fragment d'une protéine histone est choisi notamment parmi les protéines histones H2A, H3 et H4. La protéine histone est par exemple caractéristique de *L. Major, L. Infantum, L. Donovani, L. Tropica, L. Enrietti.* De manière générale, la protéine Histone peut être caractéristique d'une espèce choisie dans l'ensemble du genre *Leishmania.*

Ledit fragment peut par exemple comprendre tout ou une partie de la région N-terminale de ladite protéine histone. Ledit fragment a par exemple, une séquence polypeptidique de moins de 65 acides aminés, de préférence moins de 50 acides aminés.

Dans un exemple spécifique, la longueur de la séquence dudit fragment représente 10 à 45% de la longueur de la protéine histone.

La composition immunogène selon la demande peut comprendre alternativement au moins une protéine histone entière de *Leishmania,* en association avec le polypeptide antigénique de l'histone H2B décrit plus haut.

Ladite au moins une protéine histone entière est choisie notamment parmi H2A, H3 et H4. Elle peut être caractéristique de *L. Major, L. lnfantum, L. Donovani, L. Tropica, L. Enrietti.* De manière générale, ladite au moins une protéine histone entière peut être caractéristique d'une espèce choisie dans l'ensemble du genre *leishmania.*

Ladite composition immunogène peut être injectée comme telle, ou avec un véhicule pharmaceutiquement acceptable. La composition immunogène selon l'invention peut donc comprendre en outre un véhicule pharmaceutiquement acceptable pouvant être administré à un hôte humain ou animal. Un véhicule pharmaceutiquement acceptable pourra être facilement choisi par l'homme du métier parmi l'eau, des substances polaires telles que l'éthylène glycol, le polyéthylène glycol, ou le propylène glycol, ainsi que des substances non polaires.

Selon un mode de réalisation particulier de la demande, la composition immunogène est capable d'induire une réponse immunitaire de type cellulaire des cellules T effectrices, par exemple les cellules CD4+ lorsqu'elle est administrée à un humain ou à un animal.

Les cellules T comportent des cellules effectrices de l'immunité à médiation cellulaire (par exemple les cellules T CD4+ ou CD8+) ainsi que des cellules régulatrices (« helper » et suppressives) des réponses immunitaires. Le polypeptide selon la demande active préférentiellement les cellules effectrices de l'immunité à médiation cellulaire.

Dans la présente demande, la réponse immunitaire de type cellulaire peut être notamment une réponse immunitaire de type Th1.

Le polypeptide ou le polypeptide variant est présent, dans la composition immunogène selon la demande, dans une concentration comprise entre 0,01 µg/µl et 5 µg/µl, par exemple entre 0,01 µg/µl et 2 µg/µl, ou entre 0,4 µg/µl et 1 µg/µl.

La demande porte également sur un polypeptide consistant en un fragment de la protéine histone H2B de *Leishmania,* ledit fragment comprenant tout ou une partie de la région N-terminale de ladite protéine histone H2B.

Dans un exemple spécifique, le polypeptide est caractéristique de l'histone H2B de *Leishmania major.*

Le polypeptide peut être caractéristique de la protéine histone H2B de *L. Infantum, L. Donovani, L. Tropica, L. Enrietti.* De manière générale, le polypeptide peut être caractéristique d'une espèce choisie parmi l'ensemble du genre *Leishmania.*

Dans un exemple spécifique, le polypeptide selon la demande a une séquence polypeptidique de moins de 65 acides aminés, de préférence moins de 50 acides aminés.

Dans un exemple spécifique, le polypeptide selon la demande a une séquence polypeptidique de moins de 65 acides aminés, de préférence moins de 50 acides aminés. Le polypeptide a dans ce cadre une séquence comprenant au moins 6 acides aminés consécutifs de la partie N-terminale de l'antigène H2B considéré.

La longueur de la séquence dudit polypeptide représente par exemple 10 à 45 % de la longueur de la protéine histone.

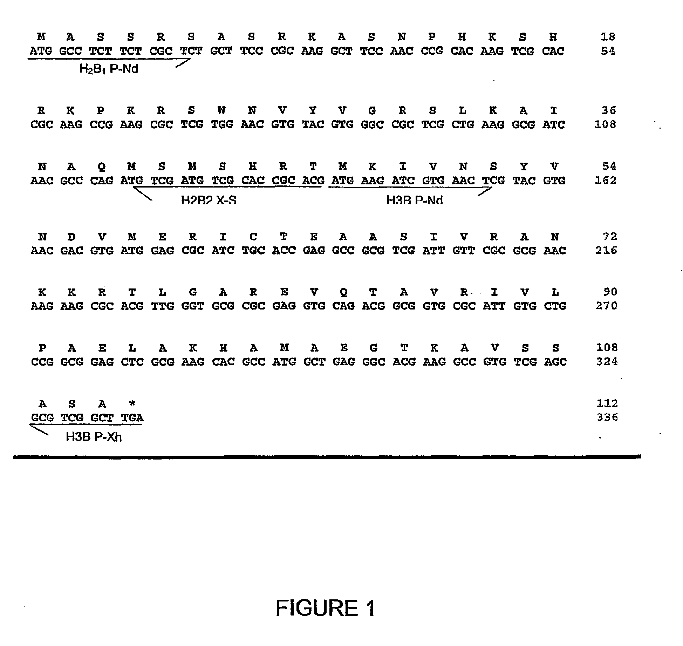

Dans un exemple spécifique, le polypeptide selon la demande comprend les 50 premiers acides aminés de la protéine histone H2B, plus particulièrement les 46 premiers acides aminés de la protéine histone H2B, comme représenté figure 1.

L'invention est relative à un polypeptide tel que défini en revendications.

L'invention est ainsi notamment relative à un polypeptide consistant en un fragment N-terminal de la protéine histone H2B de *Leishmania,* pour son utilisation dans un vaccin permettant d'obtenir une protection contre une infection par *Leishmania,* la séquence d'acides aminés dudit fragment N-terminal étant une séquence codée par :

La séquence d'acides aminés d'un polypeptide selon l'invention est par exemple la suivante: M A S S R S A S R K A S N P H K S H R K P K R S W N V Y V G R S L K A I N A Q M S M S H R T.

Dans un exemple spécifique, le polypeptide selon l'invention est un polypeptide purifié ou isolé de son environnement de production.

La demande a également pour objet un polypeptide variant conservant au moins 50% d'identité, de préférence au moins 80% d'identité avec le polypeptide selon l'invention. Ledit polypeptide variant présente une substitution, une délétion ou une addition, d'au moins un acide aminé, par rapport à la séquence dudit polypeptide et conserve les propriétés immunogènes dudit polypeptide.

Le polypeptide variant a une séquence polypeptidique de moins de 75 acides aminés, de préférence moins de 60 acides aminés.

A titre illustratif, les polypeptides variants peuvent comporter des substitutions conservatives d'acides aminés dans la séquence de l'histone H2B de *Leishmania* illustrée ci-dessus.

Un autre mode de réalisation des variants polypeptidiques fait appel à la préparation de peptides de la protéine histone H2B considérée, lesdits peptides ayant par exemple des séquences peptidiques comportant de 6 à 30, par exemple de 6 à 20 ou de 6 à 15 acides aminés, choisies à partir de la séquence N-terminale de la protéine histone.

Les peptides peuvent être utilisés en mélanges, y compris sous la forme de mixotopes. Les mélanges peptidiques comprennent par exemple au moins 2 ou au moins 5 séquences peptidiques différentes les unes des autres ou au contraire ayant des acides aminés consécutifs en commun.

Les polypeptides (y compris les peptides) de la demande et de l'invention sont préparés par toutes méthodes connues de l'homme du métier, par exemple par synthèse chimique, ou expression dans un système cellulaire, notamment bactérien, ou *in vitro* dans un système "cell free".

La demande décrit également une molécule d'acide nucléique codant le polypeptide ou le polypeptide variant selon la demande.

L'invention est relative à une molécule d'acide nucléique telle que définie en revendications.

L'invention est ainsi notamment relative à une molécule d'acide nucléique codant un polypeptide selon l'invention, pour son utilisation dans un vaccin permettant d'obtenir une protection contre une infection par *Leishmania.*

La séquence nucléotidique de ladite molécule d'acide nucléique est la suivante : ATG GCC TCT TCT CGC TCT GCT TCC CGC AAG GCT TCC AAC CCG CAC AAG TCG CAC CGC AAG CCG AAG CGC TCG TGG AAC GTG TAC GTG GGC CGC TCG CTG AAG GCG ATC AAC GCC CAG ATG TCG ATG TCG CAC CGC ACG.

La demande concerne également un vaccin permettant d'obtenir une protection contre une infection par *Leishmania.*

Le principe actif dudit vaccin comprend le polypeptide, le polypeptide variant, ou la molécule d'acide nucléique selon la demande ou conforme à l'invention. Dans un exemple spécifique, le vaccin comprend en outre un adjuvant stimulant la réponse immunitaire lorsqu'il est administré à un hôte humain ou animal, et de façon générale, ledit vaccin est préparé à partir d'une composition immunogène selon la demande, décrite ci-dessus.

Le terme « vaccin » signifie une préparation antigénique permettant de réaliser la prévention d'infections microbiennes par vaccination d'un hôte. Le vaccin selon la demande, ou conforme à l'invention peut alternativement être préparé pour conduire à une activité thérapeutique contre l'infection.

L'adjuvant favorise par exemple une réponse immunitaire de type cellulaire et dans le cas de la présente invention peut être le CpG.

Ledit vaccin peut être administré, par exemple injecté comme tel, ou avec un véhicule pharmaceutiquement acceptable. Un véhicule pharmaceutiquement acceptable pourra être choisi facilement par l'homme du métier parmi l'eau, des substances polaires telles que l'éthylène glycol, le polyéthylène glycol, ou le propylène glycol, ainsi que des substances non polaires.

Dans un exemple spécifique, le vaccin comprend au moins un autre antigène de *Leishmania* ajouté en tant que principe actif, ledit antigène ayant des propriétés immunogènes pour déclencher et/ou favoriser une réponse immunitaire, de type B et/ou T chez un hôte humain ou animal.

Le vaccin peut comprendre en outre au moins un polypeptide consistant en un fragment d'au moins une protéine histone différente de H2B de *Leishmania* ou une molécule d'acide nucléique codant pour ledit ou lesdits polypeptide(s), ou peut comprendre en outre au moins une protéine histone entière de *Leishmania,* en association avec le polypeptide antigénique de l'histone H2B décrit plus haut, ou une molécule d'acide nucléique codant pour ladite ou lesdites protéine(s) histone(s) entière(s).

Ledit fragment d'une protéine histone est choisi notamment parmi les protéines histones H2A, H3 et H4. De manière générale, ladite au moins une protéine histone peut être caractéristique d'une espèce choisie dans l'ensemble du genre *leishmania.* Ladite au moins une protéine histone est par exemple caractéristique de *L. Major, L. Infantum, L. Donovani, L. Tropica, L. Enrietti.*

Ledit fragment peut par exemple comprendre tout ou une partie de la région N-terminale de ladite protéine histone. Ledit fragment a par exemple, une séquence polypeptidique de moins de 65 acides aminés, de préférence moins de 50 acides aminés.

Dans un exemple spécifique, la longueur de la séquence dudit fragment représente 10 à 45% de la longueur de la protéine histone.

Le vaccin selon la demande, ou conforme à l'invention peut être utilisé pour prévenir ou traiter une infection par *Leishmania major* lorsqu'il est administré à un humain ou à un animal.

Le vaccin selon la demande, ou conforme à l'invention est capable d'induire une réponse immunitaire de type cellulaire des cellules T effectrices, par exemple les cellules CD4+ lorsqu'elle est administrée à un humain ou à un animal.

Les cellules T comportent des cellules effectrices de l'immunité à médiation cellulaire (par exemple les cellules T CD4+ ou CD8+) ainsi que des cellules régulatrices (« helper » et suppressives) des réponses immunitaires. Le polypeptide selon la demande ou l'invention active préférentiellement les cellules effectrices de l'immunité à médiation cellulaire.

Cette réponse immunitaire de type cellulaire peut être notamment une réponse immunitaire de type Th1.

Il est connu que le CpG est capable d'activer les monocytes, les macrophages et les cellules dendritiques. Ainsi, le CpG injecté en même temps qu'un antigène peut orienter la réponse immune spécifique vers le type Th1.

Les voies d'administration du vaccin selon l'invention sont diverses et comprennent les voies intraveineuse, orale, transdermale et nasale ; cependant les voies intramusculaire et sous-cutanée sont les plus courantes.

Le principe actif du vaccin selon la demande, ou conforme à l'invention est présent en quantité suffisante. L'expression « quantité suffisante » signifie une quantité ou une concentration en principe actif capable d'engendrer une protection immunitaire contre les Leishmanioses. Eventuellement, les directives pour la vaccination peuvent prescrire l'administration répétée de doses de vaccin.

Le polypeptide, ou le polypeptide variant ou la molécule d'acide nucléique, est présent dans le vaccin, selon la demande, ou conformément à l'invention, dans une concentration comprise entre 0,01 µg/µl et 5 µg/µl, par exemple entre 0,01 µg/µl et 2 µg/µl, ou entre 0,4 µg/µl et 1 µg/µl.

La demande décrit enfin un vecteur d'expression capable d'exprimer le polypeptide ou le polypeptide variant selon la demande ou l'invention, ledit vecteur comprenant une molécule d'acide nucléique selon la demande ou l'invention.

Le terme « vecteur d'expression » signifie le moyen par lequel des acides nucléiques, notamment de l'ADN, de l'ADN complémentaire, de l'ARN ou leur variant, sont introduits dans une cellule, particulièrement une cellule procaryote, pour exprimer ou produire une protéine déterminée.

Dans le but d'exprimer une certaine protéine ou un certain polypeptide, le vecteur d'expression peut contenir au moins un promoteur, au moins un opérateur, au moins un codon de terminaison, ainsi que toutes séquences nécessaires à une transcription efficace et à une traduction des acides nucléiques à partir du vecteur. L'ensemble de ces éléments est connu de l'homme du métier.

La demande décrit aussi des cellules hôtes, par exemple procaryotes, notamment bactériennes, transformées ou transfectées par un vecteur d'expression comprenant un polynucléotide codant pour le polypeptide selon la demande ou l'invention, dans des conditions permettant son expression.

La demande décrit aussi un plasmide pET-CH2B contenant la séquence de la partie carboxy-terminale de la protéine H2B, déposé à la Collection Nationale de Cultures de Micro-organismes (CNCM) sous le n° I-3362, le 24 février 2005.

La demande décrit aussi un plasmide pET-NH2B contenant la séquence de la partie amino-terminale divergente de la protéine H2B, déposé à la Collection Nationale de Cultures de Micro-organismes (CNCM) sous le n° I-3363, le 24 février 2005. L'invention peut mettre en oeuvre ce vecteur.

La demande décrit aussi un plasmide pET-H2B contenant la séquence de la protéine H2B, déposé à la Collection Nationale de Cultures de Micro-organismes (CNCM) sous le n° I-3361, le 24 février 2005.

### LEGENDE DES FIGURES

**Figure 1*****:** La séquence nucléotidique et protéique de l'histone H2B de L. major.* Les séquences repérées par les notations H2B1 P-ND up, H2B2 X-S rev, H3B P-Nd up et H3B P-Xh rev correspondent aux amorces d'amplification des deux parties h2bΔC65(1-138pb) (N-terminale) et h2bΔN46(139-333pb) (C-terminale) de la protéine.
**Figure 2** *: Analyse de l'expression et de la purification des protéines histones recombinantes H2BΔC65 (A), H2BΔN46 (B) et H2B (C) de L. major sur gel de polyacrylamide à* 18% *après coloration au bleu de Commassie.*
   **Puits 1 :** extraits brut de bactéries BL21 transformées par les plasmides recombinants respectifs et non induites à l'IPTG.
   **Puits 2 :** extrait brut de bactéries BL21 transformées par les plasmides recombinants respectifs et induites à l'IPTG.
   **Puits 3 :** protéines histones recombinantes purifiées sur colonne de Nickel (H2B, H2BΔC65 et H2BΔN46 de *L. major).*
   **Puits PM :** marqueurs de poids moléculaires.

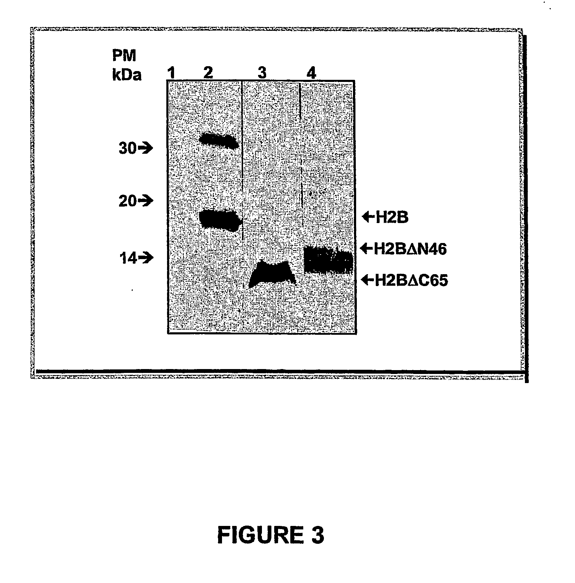

**Figure 3****:** *Analyse par immunoblot de la réactivité des anticorps polyclonaux anti-H2B de lapin vis-à-vis des protéines histones recombinantes H2B, H2BΔC65 et H2BΔN46 de L. major après purification sur colonne de Nickel.*
   **Puits 1:** Extrait brut de bactéries BL21 transformées par les plasmides recombinants respectifs et non induites à l'IPTG.
   **Puits 2, 3, 4:** Protéines histones recombinantes purifiées sur colonne de Nickel (H2B, H2BΔC65 et H2BΔN46 de *L. major).*

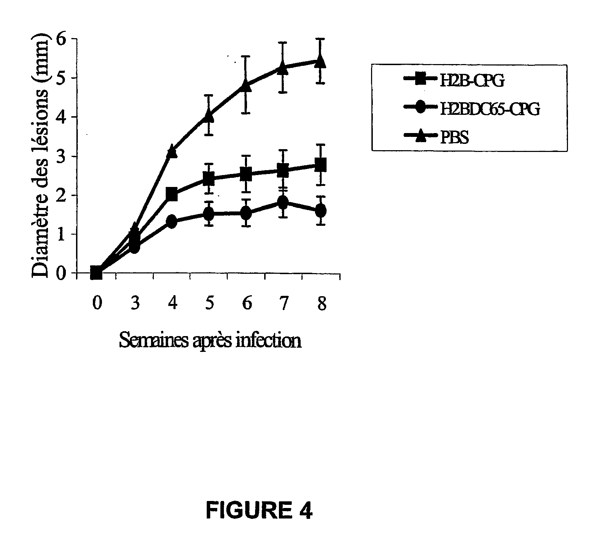

**Figure 4****:** Représentation graphique de l'évolution du diamètre des lésions chez les souris BALB/c contrôle (PBS) ou immunisées par H2B (H2B-CpG) ou juste la partie amino terminale de H2B (H2BDC65-CpG) en présence de 25 ug de CpG/injection, utilisé comme adjuvant. Les souris ont été infectées, 30 jours après le rappel, par 10⁵ promastigotes de *L. major.* Les valeurs représentent la moyenne des lésion exprimées en mm +/- l'écart type.

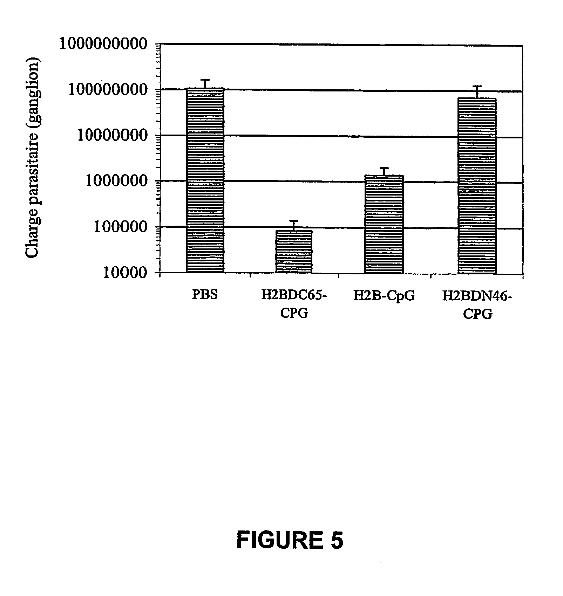

**Figure 5****:** Détermination de la moyenne du nombre de parasites par ganglion par la technique de dilutions limites chez les différents lots des souris immunisés deux fois en sous cutané, au niveau du coussinet plantaire par H2B+CpG, H2BΔC65+CpG et H2BΔN46+CpG. La quantification a été réalisée 8 semaines après le challenge par 10⁶ promastigotes de *L. major.* Les résultats sont exprimés par la moyenne du nombre de parasites par ganglion de la dilution la plus forte dans laquelle au moins un promastigote de *L. major* est viable.

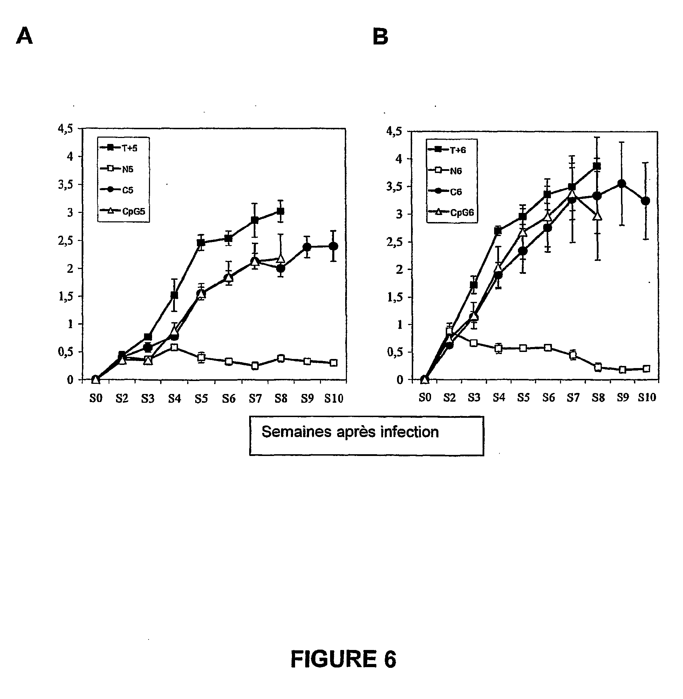

**Figure 6*****:*** Représentation graphique de l'évolution des moyennes des diamètres des lésions (mm) chez les souris BALB/c vaccinées ou non et après un challenge par *L.major* à raison de 10⁵ (A) ou 10⁶ (B) promastigotes métacycliques.
   T+5 ou 6 : groupe contrôle (PBS) après un challenge avec 10⁵ (5) ou 10⁶ (6) parasites.
   N+5 ou 6 : Souris ayant reçu la région amino terminale de H2B
   C+5 ou 6 : Souris ayant reçu la région carboxy terminale de H2B
   CpG+5 ou 6 : Souris ayant reçu seulement l'adjuvant (CpG)
   Les valeurs représentent la moyenne des lésions exprimées en mm +/-l'écart type.

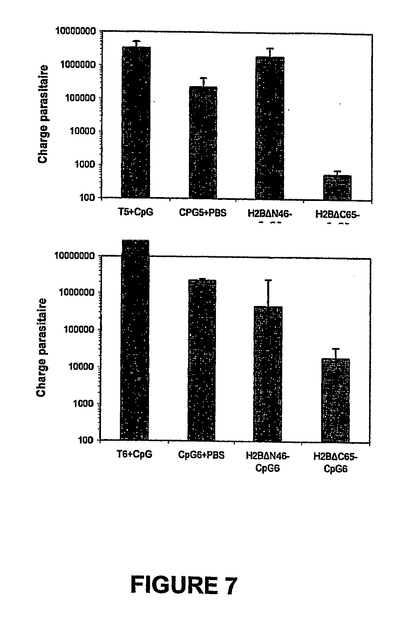

**Figure 7****:** Détermination de la charge parasitaire par la techniques de dilutions limites chez les différents lots des souris immunisés trois fois en sou cutané, au niveau du coussinet plantaire par H2BΔN46+CpG, H2BΔC65+CpG ainsi que les contrôles CpG. La quantification a été réalisée 10 semaines après le challenge par 10⁵ ou 10⁶ promastigotes de *L. major.* Les résultats sont exprimés comme dans la figure 5.

### EXEMPLES

### Matériel et Méthodes

### Parasites et conditions de culture

La souche parasitaire MHOM/TN/94/GLC94 (GLC94) isolée d'une lésion humaine de leishmaniose cutanée zoonotique (LCZ) et a été utilisée dans le présent travail (Louzir et coll, 1998). Cet isolat appartient à l'espèce *L. major,* zymodème MON25 (Kebaier et coll, 2001). Les parasites ont été cultivés sur milieu NNN à 26°C et progressivement transférés dans du RPMI (SIGMA, St Louis, MO) contenant 2mM de L-Glutamine, 100U/ml de pénicilline, 100µg/ml de streptomycine et 10% de Sérum de Veau Foetal inactivé (milieu complet). Les promastigotes, pris en phase logarithmique de croissance, sont ajustés à 10⁶ parasites/ml dans un volume constant de milieu complet puis incubés à 26°^{c}. La phase stationnaire de croissance est atteinte après 4 à 6 jours de culture avec des densités de parasites allant de 3.10⁷ à 8.10⁷ parasites/ml. Les promastigotes metacycliques sont ensuite purifiés sur un gradient de Ficoll 5%-20%, lavés, comptés puis injectés aux souris.

### Expression et purification des différentes protéines recombinantes dans les bactéries E. coli BL21

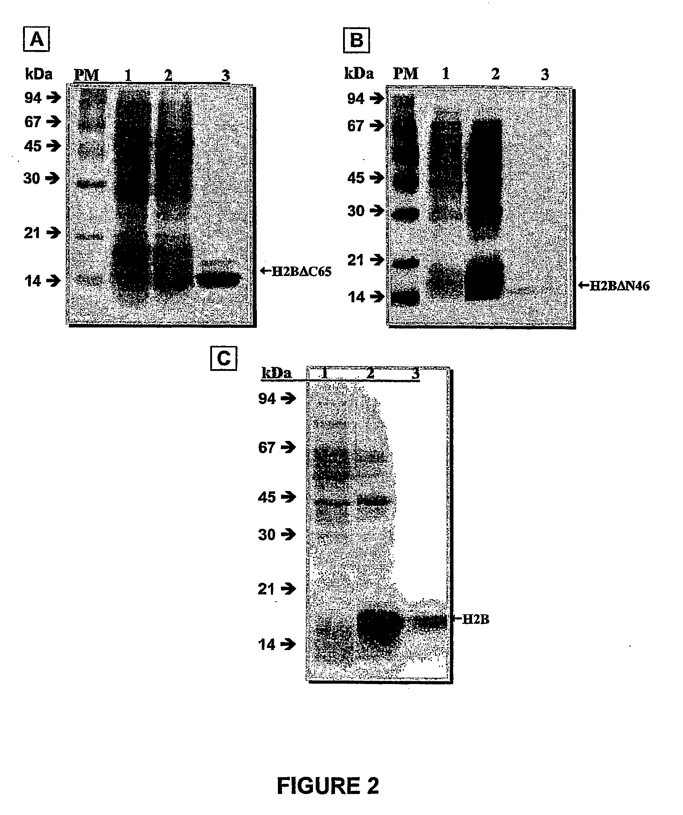

Les séquences codantes pour la protéine histone H2B entière (339pb), la partie amino-terminale (1-46aa) divergente H2BΔC65 (138pb) et la partie carboxy-terminale (47-111aa) conservée H2bΔN46 (198pb) ont été clonées dans le vecteur d'expression bactérien pET-22b (Novagen) (figure 1). Les bactéries *E*. *coli* BL21 contenant les plasmides recombinants (pET-22b-H2B, pET-H2BΔC65 et pET- H2BΔN46) ont été cultivées dans du milieu LB puis la synthèse de la protéine recombinante a été induite en présence de 1mM d'isopropyl-1-thio-β-D-galactopyranoside (IPTG) pendant 4 heures. Les protéines recombinantes ont été purifiées par chromatographie d'affinité sur une colonne de nickel (Ni²⁺) (Amersham-Pharmacia). La pureté des protéines produites a été vérifiée par SDS-PAGE (figure 2).

Le plasmide pET-CH2B contenant la séquence de la partie carboxy-terminale de la protéine H2B a été déposé à la Collection Nationale de Cultures de Micro-organismes (CNCM) le 24 février 2005 sous le n° I-3362.

Description du pET-CH2B : Le plasmide pET-CH2B a été obtenu par clonage dans le plasmide pET-22b+ (Novagen), de la séquence d'ADN complémentaire codant pour la protéine histone H2B tronquée des 46 premiers acides aminés (H2BΔN46) de *Leishmania major.* Le gène H2BΔN46 a été cloné au niveau des sites de restriction Ndel et XhoI de pET-22b+. Des bactéries recombinantes *E. coli* XL1-blue ont été transformées avec pET-CH2B. Elles sont résistantes à la tétracycline et à l'ampiciline. Les gènes de résistance à la tétracycline et à l'ampiciline sont portés par les bactéries XL1-blue et le plasmide pET-22b+ recombinant.

Le plasmide pET-NH2B contenant la séquence de la partie amino-terminale divergente de la protéine H2B a été déposé à la Collection Nationale de Cultures de Micro-organismes (CNCM) le 24 février 2005 sous le n° I-3363.

Description du pET-NH2B : Le plasmide pET-NH2B a été obtenu par clonage dans le plasmide pET-22b+ (Novagen), de la séquence d'ADN complémentaire codant pour la protéine histone H2B tronquée des 65 derniers acides aminés (H2BΔC65) de Leishmania major.
Le gène H2BΔC65 a été cloné au niveau des sites de restriction Ndel et Xhol. Des bactéries recombinantes *E. coli* XL1-blue ont été transformées avec pET-NH2B. Elles sont résistantes à la tétracycline et à l'ampiciline. Les gènes de résistance à la tétracycline et à l'ampiciline sont portés par les bactéries XL1-blue et le plasmide pET-22b+ recombinant.

Le plasmide pET-H2B contenant la séquence de la protéine H2B a été déposé à la Collection Nationale de Cultures de Micro-organismes (CNCM) le 24 février 2005 sous le n° I-3361.

Description du pET-H2B : Le plasmide pET-H2B a été obtenu par clonage dans le plasmide pET-22b+ (Novagen), de la séquence d'ADN complémentaire codant pour la protéine histone H2B entière de *Leishmania major.* Le gène H2B a été cloné au niveau des sites de restriction Ndel et Xhol.
Des bactéries recombinantes *E. coli* XL1-blue ont été transformées avec pET-H2B. Elles sont résistantes à la tétracycline et à l'ampiciline. Les gènes de résistance à la tétracycline et à l'ampiciline sont portés par les bactéries XL1-blue et le plasmide pET-22b+ recombinant.

Le plasmide pET-H2B est encore désigné pET-22b-H2B dans ce qui suit. Le plasmide pET-CH2B est encore désigné pET-H2BΔN46 dans ce qui suit.
Le plasmide pET-NH2B est encore désigné pET-H2BΔC65 dans ce qui suit.

Les conditions de culture des bactéries transformées sont les suivantes :

### Milieu de culture préconisé

Les bactéries recombinantes sont cultivées soit en milieu de culture liquide soit en milieu de culture solide.

Le milieu de culture liquide, Luria Broth (LB) est composé de: 1% (poids/volume) de Bactotryptone, 0,5% (p/v) d'extraits de levure et de 1 % (p/v) de NaCl. Le pH du milieu doit être réglé à 7. Ce milieu est préconisé pour la multiplication des plasmides pET-22b+ contenant le gène H2BΔC65 (ou contenant le gène H2BΔN46 ou le gène H2B).

Le milieu de culture solide, Luria Broth Agar (LB-Agar) est constitué de 1 % (poids/volume) de Bactotryptone, 0,5% (p/v) d'extraits de levure, de 1% (p/v) de NaCl et de 1,5% (p/v) d'agar. Ce milieu est essentiellement préconisé lors des cultures réalisées à partir de bactéries recombinantes congelées à -0,80°c.

Ces deux milieux de culture sont stérilisés à 120 °c pendant 20 minutes juste après leur préparation.

### Conditions d'ensemencement

Les bactéries recombinantes congelées à -80 °c sont tout d'abord étalées sur une boite contenant du milieu de culture solide LB-Agar supplémenté de 15µg/ml de tétracycline et de 50µg/ml d'ampiciline pendant 18 heures à 37°c puis une colonie isolée est prélevée et incubée dans 5ml de LB additionné des même antibiotiques. La culture est réalisée pendant 18 heures à 37°c.

Incubation (température, atmosphère agitation illumination)

La température de culture est toujours réalisée à 37°c sous une agitation de 200tr/min (appareil SANYO Orbital Incubator). La culture est réalisée sous une atmosphère ambiante et une illumination naturelle.

### Protocole de vaccination et challenge des souris

Deux protocoles expérimentaux ont été réalisés, dans le premier protocole, les souris ont été injectées deux fois à 15 jours d'intervalle par des différentes préparations vaccinales (tableau 1). Le deuxième protocole a été utilisé pour la confirmation et l'optimisation de certains résultats (voir le détail dans le tableau 2).

Toutes les injections ont été réalisées par voie sous-cutanée, au niveau du coussinet plantaire de la patte arrière droite dans un volume final de 50µl par injection. Quatre semaines après le dernier rappel, les souris ont reçu une injection des parasites *L. major* (promastigotes métacycliques) au niveau de coussinet plantaire de la patte arrière droite. Deux quantités de parasites ont été utilisées soit, 10⁵ et 10⁶ parasites/injection. Le suivi de l'évolution de la maladie expérimentale a été fait par la mesure hebdomadaire du diamètre de la lésion.

**Tableau 1:**

| Expérience 1, protocole expérimental d'immunisation et de challenge des souris. | | | | |
|---|---|---|---|---|
| **N° groupe (nb souris)** | Préparation vaccinales | Doses | **Voie d'injection** | **Challenge** |
| 1 (4 souris) | **PBS** | 50 µl | Sous-cutanée | 10⁵ parasites |
| 2 (4 souris) | H2B+CpG | 25µg/20µg | Sous-cutanée | 10⁵ parasites |
| 3 (4 souris) | H2B+nCpG | 25µg/µg | Sous-cutanée | 10⁵ parasites |
| 4 (4 souris) | H2BΔC65+CpG | 25µg/20µg | Sous-cutanée | 10⁵ parasites |
| 5 (4 souris) | H2BΔC65+nCpG | 25µg/20µg | Sous-cutanée | 10⁵ parasites |
| 6 (4 souris) | H2BΔN46+CpG | 25µg/20µg | Sous-cutanée | 10⁵ parasites |
| 7 (4 souris) | H2BΔN46+nCpG | 25µg/20µg | Sous-cutanée | 10⁵ parasites |
| 8 (4 souris) | CpG | 20µg | Sous-cutanée | 10⁵ parasites |
| 9 (4 souris) | nCpG | 20µg | Sous-cutanée | 10⁵ parasites |
| 10 (4 souris) | H2B | 25µg | Sous-cutanée | 10⁵ parasites |
| 11 (4 souris) | H2BΔC65 | 25µg | Sous-cutanée | 10⁵ parasites |
| 12 (4 souris) | H2BΔN46 | 25µg | Sous-cutanée | 10⁵ parasites |

**Tableau 2:**

| Expérience 2, protocole expérimental d'immunisation et de challenge des souris. | | | | |
|---|---|---|---|---|
| **N° groupe (nb souris)** | **Préparations vaccinales** | **Doses/volumes** | ***Voie d'injection*** | ***Challenge*** |
| 1 (6 souris) | CpG | 50µg | Sous-cutanée | 10⁵ parasites |
| 2 (6 souris) | CpG | 50µg | Sous-cutanée | 10⁶ parasites |
| 3 (6 souris) | H2BΔC65+CpG | 20µg/50µg | Sous-cutanée | 10⁵ parasites |
| 4 (6 souris) | H2BΔC65+CpG | 20µg/50µg | Sous-cutanée | 10⁶ parasites |
| 5 (6 souris) | H2BΔN46+CpG | 20µg/50µg | Sous-cutanée | 10⁵ parasites |
| 6 (6 souris) | H2BΔN46+CpG | 20µg/50µg | Sous-cutanée | 10⁶ parasites |
| 7 (6 souris) | PBS | 50 µl | Sous-cutanée | 10⁵ parasites |
| 8 (6 souris) | PBS | 50 µl | Sous-cutanée | 10⁶ parasites |

### Charge parasitaire

La quantification des parasites a été estimée au niveau des sites lésionnels en utilisant une variante de la technique des dilutions limites (Titus RG, Marchand M, Boon T, Louis JA. A limiting dilution assay for quantifying Leishmania major in tissues of infected mice. Parasite Immunol 1985; 7:545-55). Brièvement, la patte infectée a été coupée, homogénéisée dans un volume de 5ml de milieu RPMI additionné d'antibiotiques puis une série de dilutions de 1 :10 jusqu'à 1 :10¹¹ ont été faites en quatre exemplaires chacune dans une plaque de microtitration (96 puits, Nunc, Roskilde, Denmark) contenant du milieu RPMI additionné de 100 U/ml de pénicilline, 100 µg/ml streptomycine, 2mM L-glutamine et 10% de sérum de veau décomplémenté. Les plaques ont été incubées à 26°C pendant au moins 15 jours. La recherche des parasites vivants se fait sous microscope inversé. La charge parasitaire est exprimée par la moyenne du négatif du log de la dernière dilution dans laquelle des parasites mobiles ont été détectés.

### Résultats

### Production et purification des différentes protéines recombinantes

La protéine H2B entière (111aa), la partie divergente de H2B située au niveau des 46 premiers aa et la partie conservée de H2B localisée entre les acides aminés 47 à 111 ont d'abord été exprimées en utilisant le système d'expression procaryote pET puis purifiées par chromatographie d'affinité sur une colonne de nickel (figure 2). L'anticorps polyclonal anti-H2B obtenu après immunisation de souris BALB/c par la protéine recombinante H2B entière reconnaît spécifiquement, par immunoblot, les protéines recombinantes H2B, H2BΔC65 et H2BΔN46 (figure 3).

### Evaluation clinique et parasitologique de l'effet de la vaccination des souris BALB/c par la protéine H2B entière ou mutée suite à un challenge virulent par L. major

Des expériences préliminaires faites chez des souris BALB/c vaccinées par la protéine H2B seule ou en présence d'adjuvant (deux injections de 20 mg de protéine +/- 25 µg de CpG à 15 jours d'intervalle) ont montré que ces souris étaient capables, un mois après la dernière immunisation, de produire de grandes quantités d'IFN-y quand leurs cellules ganglionnaires (drainant les sites d'injection) sont re-stimulées *in vitro* avec la protéine H2B. Les quantités d'IL-10 ou IL-4 sont très faibles ou non détectables. Ces résultats suggèrent que la protéine H2B a la capacité d'induire, chez les souris BALB/c, une réponse Th1 spécifique.

La capacité des différentes préparations vaccinales à induire une protection dans le modèle murin de la leishmaniose expérimentale a été évaluée chez les souris sensibles BALB/c en utilisant deux expériences différentes et complémentaires.

### Expérience 1

Des groupes de souris BALB/c (4 souris par groupe), femelles âgées de 6 à 8 semaines, ont été injectés deux fois à 15 jours d'intervalle par différentes préparations vaccinales (tableau 1). Les protéines recombinantes testées ont été soit adjuvées au CpG soit avec un contrôle négatif du CpG, le non CpG (nCpG). Le CpG est un oligodexoynucléotide d'ADN non méthylé qui possède un motif particulier de CpG. Il a été clairement démontré dans la littérature que l'adjuvant CpG est capable d'activer les monocytes, les macrophages et les cellules dendritiques. Ainsi, le CpG injecté en même temps qu'un antigène peut orienter la réponse immune spécifique vers le type Th1 (Pour revue, Klinman et coll, 2004).
Deux immunisations ont donc été réalisées par voie sous cutanée au niveau du coussinet plantaire de la patte arrière droite. Un mois plus tard, les différents groupes de souris BALB/c ont été infectés par 2*10⁵ promastigotes métacycliques de la souche parasitaire virulente de *L. major* (GLC94) au niveau du coussinet plantaire de la patte arrière gauche.
Le résultat de l'évolution de la lésion dans chaque lot de souris immunisées et infectées a été exprimé par la moyenne de la taille des lésions obtenues chez les souris du même groupe. Après un suivi clinique de huit semaines, seuls deux lots de souris montraient un ralentissement très significatif des lésions expérimentales : il s'agit des souris immunisées par la protéine *H2B* entière en présence de CpG (H2B+CpG) et des souris immunisées par la partie amino-terminale de H2B en présence de CpG (H2BΔC65+CpG). Les différences étaient très significatives avec tous les autres groupes, incluant le groupe de souris contrôle (p<0,001 à partir de la semaine 5) (figure 4). Il est très intéressant de souligner que les souris immunisées par la partie amino-terminale de H2B en présence de CpG étaient plus résistantes que celles immunisées, dans les mêmes conditions, par la protéine entière (p<0,05, à partir de la semaine 5) quand elles étaient ensuite infectées par *L. major.* Par ailleurs tous les autres groupes développaient une maladie expérimentale similaire à celle observée avec les souris contrôles (injectées par le PBS).
En conclusion, les résultats obtenus montrent que la partie NH2-teminale de la protéine H2B et la protéine H2B entière en présence de l'adjuvant CpG sont capables d'induire un effet protecteur chez des souris BALB/c après un challenge virulent par *L. major.* De plus, la comparaison des courbes d'évolution de lésions entre ces deux lots de souris montre une meilleure résistance (statistiquement significative) des souris vaccinées par H2BΔC65+CpG par rapport aux souris vaccinées par H2B+CpG.

### Evaluation de la charge parasitaire

La détermination de la charge parasitaire dans le ganglion poplité drainant la lésion représente un facteur déterminant dans l'évaluation de vaccin anti-leishmanien (Kébaier et al; 2001, « Heterogeneity of wild Leismania major isolates in experimental murine pathogenicity and specific immune response, Infect. Immun. 2001, Aug, 69(8) : 4906-15). La charge parasitaire a été évaluée (8 semaines après infection expérimentale) uniquement chez les groupes de souris ci-dessous :
- Souris contrôle (PBS)
- Souris vaccinées par H2B+CpG
- Souris vaccinées par H2BΔC65+CpG
- Souris vaccinées par H2BΔN46+CpG

Dans la figure 5 sont représentés les résultats de la charge parasitaire analysée dans le ganglion drainant le site d'injection des parasites. Il existe une très bonne corrélation entre la charge parasitaire et la sévérité des lésions expérimentales induites chez les différents groupes de souris. Les souris vaccinées par H2BΔN46, non protégées, avaient une charge parasitaire similaire à celle retrouvée chez les souris contrôle. En revanche, les deux groupes de souris ayant reçu H2B ou la partie amino-terminale (H2BΔC65) présentaient une charge parasitaire très nettement inférieure à celle des groupes contrôles (100 à 1000 fois inférieure). Il est intéressant de souligner que les souris immunisées par la partie amino-terminale de H2B avaient une charge parasitaire moyenne au moins 10 fois inférieure à celle retrouvée chez les souris immunisées par la protéine entière (H2B). Ces résultats confirment la supériorité de la partie divergente (amino-terminale) par rapport à la protéine H2B entière dans sa capacité à conférer une protection des souris sensibles BALB/c contre un challenge virulent par *L. major.*

### Expérience 2

Dans le but de confirmer les résultats en utilisant notamment 10 fois plus de parasites pour le challenge, les inventeurs se sont intéressés aux protéines tronquées de H2B (H2BΔC65 et H2BΔN46) en utilisant une concentration plus élevée de CpG (50µg/injection) et trois immunisations à 21 jours d'intervalle. De plus deux groupes contrôles ont été utilisés (un groupe ayant reçu seulement le CpG et l'autre seulement le tampon PBS). Un mois après la dernière dose de vaccin, les souris ont été infectées par deux doses de parasites (soit 10⁵ ou 10⁶ parasites (promastigotes métacycliques)/injection). Le protocole expérimental est détaillé dans le tableau 2.

Les figures 6 et 7 montrent clairement que seules les souris immunisées par la partie divergente de la protéine H2B (H2BΔC65) résistaient à l'infection expérimentale par *L. major* même après utilisation de 10⁶ promastigotes métacycliques pour le challenge. Il existe une parfaite corrélation entre la résistance clinique et la charge parasitaire réalisée à la fin du protocole (10 semaines après infection).
Ces résultats confirment ceux obtenus avec la première expérience et suggèrent que le deuxième protocole d'immunisation confère une meilleure protection des souris BALB/c contre l'infection par *L. major.*

### Discussion

Selon les chiffres de l'Organisation Mondiale de la santé (OMS), les leishmanioses menacent dans 88 pays, 350 millions de personnes sont exposées aux risques de piqûres de phlébotomes, insectes vecteurs, et on estime à 12 millions le nombre d'individus atteints. Dans sa forme la plus grave, cette maladie tue. Le fait que la majorité des individus ayant développé une leishmaniose, ou tout simplement une infection asymptomatique, résistent à la réinfection, justifie les recherches pour le développement de vaccins. Cependant, deux problèmes majeurs restent posés : la meilleure connaissance de la nature précise des mécanismes effecteurs de la résistance ainsi que la nature des antigènes parasitaires et l'adjuvant à utiliser. Il n'existe pas actuellement de vaccin à usage humain. Les vaccins de première génération, les seuls à avoir été testé chez l'homme, ont montré leur inefficacité (Sharifi et coll, 1998 ; Khalil et coll, 2000; De Luca et coll, 2001; Armijos et coll, 2004).

Les expériences rapportées ici permettent d'identifier et d'évaluer de nouveaux candidats vaccins contre les leishmanioses.

La protéine histone H2B *L. infantum* a été initialement sélectionnée dans le laboratoire comme protéine parasitaire de faible poids moléculaire capable d'induire une réponse Th1 chez l'homme. Des données plus récentes ont suggéré le rôle de cette protéine comme candidat vaccin (Melby, 2000 ; Probst et coll 2001 ; Iborra et coll, 2004).

Les travaux décrits ici reposent sur l'analyse comparée de l'immunogénicité et du rôle protecteur des deux régions (divergente et conservée) de la protéine recombinante H2B de Leishmania ainsi que de la protéine entière. Les résultats montrent clairement que la partie amino-terminale (divergente) de H2B adjuvée au CpG a conféré une très bonne protection des souris sensibles BALB/c contre un challenge virulent par *L. major.* A l'inverse, la partie conservée (carboxy-terminale), injectée dans les mêmes conditions n'a eu aucun effet protecteur. Il est extrêmement intéressant de souligner que la protéine H2B entière conférait une protection intermédiaire. L'ensemble des résultats suggère que la partie amino-terminale (divergente) de H2B puisse constituer un bon candidat vaccin contre les leishmanioses.

### Références

Alimohammadian MH, Khamesipour A, Darabi H, et al. The role of BCG in human immune responses induced by multiple injections of autoclaved Leishmania major as a candidate vaccine against leishmaniasis. Vaccine 2002; 21:174-80.
Armijos RX, Weigel MM, Calvopina M, Hidalgo A, Cevallos W, Correa J. Safety, immunogenecity, and efficacy of an autoclaved Leishmania amazonensis vaccine plus BCG adjuvant against New World cutaneous leishmaniasis. Vaccine. 2004 Mar 12;22(9-10):1320-6.
Belkaid Y, Piccirillo CA, Mendez S, Shevach EM, Sacks DL: CD4+CD25+ regulatory T cells control Leishmania major persistence and immunity. Nature 2002; 420:502-507.
Brodskyn C, Beverley SM, Titus RG. Virulent or avirulent (dhfr-ts-) Leishmania major elicit predominantly a type-1 cytokine response by human cells in vitro. Clin Exp Immunol. 2000;119(2):299-304.
Campbell K, Diao H, Ji J, Soong L. DNA immunization with the gene encoding P4 nuclease of Leishmania amazonensis protects mice against cutaneous Leishmaniasis. Infect Immun. 2003;71(11):6270-8.
Campos-Neto A, Webb JR, Greeson K, Coler RN, Skeiky YA, Reed SG. Vaccination with plasmid DNA encoding TSA/LmSTI1 leishmanial fusion proteins confers protection against Leishmania major infection in susceptible BALB/c mice. Infect Immun. 2002;70(6):2828-36.
Coler RN, Skeiky YA, Bernards K, Greeson K, Carter D, Cornellison CD, Modabber F, Campos-Neto A, Reed SG. Immunization with a polyprotein vaccine consisting of the T-Cell antigens thiol-specific antioxidant, Leishmania major stress-inducible protein 1, and Leishmania elongation initiation factor protects against leishmaniasis. Infect Immun. 2002; 70(8):4215-25.
Davies CR, Lianos-Cuentas EA, Pyke SDM et al. Cutaneous leishmaniasis in the Peruvian Andes: an epidemiological study of infection and immunity. Epedimiol Infect 1995; 114: 297-318.
De Luca PM, Mayrink W, Pinto JA, et al. A randomized double-blind placebo-controlled trial to evaluate the immunogenicity of a candidate vaccine against American tegumentary leishmaniasis. Acta Trop 2001; 80:251-60.
Desjeux P. Leishmaniasis. Public health aspects and control. Clin Dermatol 1996; 14:417-23.
Dumonteil E, Maria Jesus RS, Javier EO, Maria del Rosario GM. DNA vaccines induce partial protection against Leishmania mexicana. Vaccine. 2003;21(17-18):2161-8.
Gavin, M. and Rudensky, A. Control of immune homeostasis by naturally arising regulatory CD4+ T cells. Curr. Opin. Immunol 2003; 15(6):690-6.
Guirges SY. Natural and experimental re-infection of man with oriental score. Ann Trop Med Parasitol 1971; 65 :197-205.
Gurunathan S, Sacks DL, Brown DR, Reiner SL, Charest H, Glaichenhaus N, Seder RA. Vaccination with DNA encoding the immunodominant LACK parasite antigen confers protective immunity to mice infected with Leishmania major. J Exp Med. 1997;186(7):1137-47.
Handman E, Symons FM, Baldwin TM, Curtis JM, Scheerlinck JP. Protective vaccination with promastigote surface antigen 2 from Leishmania major is mediated by a TH1 type of immune response. Infect Immun. 1995;63(11):4261-7.
Iborra S, Soto M, Carrion J, Nieto A, Fernandez E, Alonso C, Requena JM. The Leishmania infantum acidic ribosomal protein P0 administered as a DNA vaccine confers protective immunity to Leishmania major infection in BALB/c mice. Infect Immun. 2003;71(11):6562-72.
Iborra S, Soto M, Carrion J, Alonso C, Requena JM. Vaccination with a plasmid DNA cocktail encoding the nucleosomal histones of Leishmania confers protection against murine cutaneous leishmaniosis. Vaccine. **2004**;22(29-30):3865-76.
Kébaïer C, Louzir H, Chenik M, Ben Salah A, Dellagi K. Heterogeneity of wild Leishmania major isolates in experimental murine pathogenicity and specific immune response. Infection and Immunity. 2001. 69(8), 4906-4915.
Khalil, EA, El Hassan AM, Zijlstra EE, et al. Autoclaved Leishmania major vaccine for prévention of visceral leishmaniasis: a randomised, double-blind, BCG-controlled trial in Sudan. Lancet 2000; 356:1565-9.
Klinman DM. Use of CpG oligodeoxynucleotides as immunoprotective agents. Expert Opin Biol Ther. 2004;4(6):937-46.
   WHO Expert Commitee. Control of the Leishmaniasis. WHO Tech Rep Ser 1990; 793: 0-158.
Louzir H, Melby PC, Ben Salah A, Marrakchi H, Aoun K, Ben Ismail R, Dellagi K. Immunologic determinants of disease evolution in localized cutaneous leishmaniasis due to Leishmania major. Journal of Infectious Diseases. 1998. 177(6), 1687-1695.
Maalej IA, Chenik M, Louzir H, Ben Salah A, Bahloul C, Amri F, Dellagi K. Comparative evaluation of ELISAs based on ten recombinant or purified Leishmania antigens for the serodiagnosis of Mediterranean visceral leishmaniasis. Am J Trop Med Hyg. 2003;68(3):312-20.
Melby PC, Ogden GB, Flores HA, Zhao W, Geldmacher C, Biediger NM, Ahuja SK, Uranga J, Melendez M. Identification of vaccine candidates for experimental visceral leishmaniasis by immunization with sequential fractions of a cDNA expression library. Infect Immun. 2000;68(10):5595-602.
Probst P, Stromberg E, Ghalib HW, Mozel M, Badaro R, Reed SG, Webb JR. Identification and characterization of T cell-stimulating antigens from Leishmania by CD4 T cell expression cloning. J Immunol. 2001;166(1):498-505.
Ramiro MJ, Zarate JJ, Hanke T, Rodriguez D, Rodriguez JR, Esteban M, Lucientes J, Castillo JA, Larraga V. Protection in dogs against visceral leishmaniasis caused by Leishmania infantum is achieved by immunization with a heterologous prime-boost regime using DNA and vaccinia recombinant vectors expressing LACK. Vaccine. 2003;21(19-20):2474-84.
Sharifi I, Reza A, Aflatonian MR, et al. Randomised vaccine trial of single dose of killed Leishmania major plus BCG against anthroponotic cutaneous leishmaniasis in Bam, Iran. Lancet 1998; 351:1540-3.
Skeiky YA, Coler RN, Brannon M, Stromberg E, Greeson K, Crane RT, Webb JR, Campos-Neto A, Reed SG. Protective efficacy of a tandemly linked, multi-subunit recombinant leishmanial vaccine (Leish-111f) formulated in MPL adjuvant. Vaccine. 2002; 10;20(27-28):3292-303.
Skeiky YA, Kennedy M, Kaufman D, Borges MM, Guderian JA, Scholler JK, Ovendale PJ, Picha KS, Morrissey PJ, Grabstein KH, Campos-Neto A, Reed SG. LeIF: a recombinant Leishmania protein that induces an IL-12-mediated Th1 cytokine profile. J Immunol. 1998; 161(11):6171-9.
Soto M, Requena JM, Quijada L, Perez MJ, Nieto CG, Guzman F, Patarroyo ME, Alonso C. Antigenicity of the Leishmania infantum histones H2B and H4 during canine viscerocutaneous leishmaniasis. Clin Exp Immunol. 1999;115(2):342-9.
Spitzer N, Jardim A, Lippert D, Olafson RW. Long-term protection of mice against Leishmania major with a synthetic peptide vaccine. Vaccine. 1999 Mar 17;17(11-12):1298-300.
Streit JA, Recker TJ, Filho FG, Beverley SM, Wilson ME. Protective immunity against the protozoan Leishmania chagasi is induced by subclinical cutaneous infection with virulent but not avirulent organisms. J Immunol. 2001;166(3):1921-9.
Wolffe AP. Transcriptional regulation in the context of chromatin structure. Essays Biochem. 2001;37:45-57.

## Revendications

1. Polypeptide consistant en un fragment N-terminal de la protéine histone H2B de *Leishmania,* pour son utilisation dans un vaccin permettant d'obtenir une protection contre une infection par *Leishmania,* la séquence d'acides aminés dudit fragment N-terminal étant une séquence codée par :

2. Polypeptide selon la revendication 1, pour son utilisation dans un vaccin selon la revendication 1 dont la séquence d'acides aminés est : MASSRSASRKASNPHKSHRKPKRSWNVYVGRSLKAINAQMSMSHRT.

3. Polypeptide selon l'une quelconque des revendications précédentes, pour son utilisation dans un vaccin selon la revendication 1 **caractérisé en ce que**, dans ladite utilisation, ledit polypeptide est utilisé à titre d'agent induisant ou favorisant l'activation de cellules effectrices de l'immunité à médiation cellulaire.

4. Polypeptide selon l'une quelconque des revendications précédentes, pour son utilisation dans un vaccin selon la revendication 1 **caractérisé en ce que**, dans ladite utilisation, ledit polypeptide est utilisé à titre d'agent induisant ou favorisant une réponse immunitaire de type Th1.

5. Polypeptide selon l'une quelconque des revendications précédentes, pour son utilisation dans un vaccin selon la revendication 1 **caractérisé en ce que**, dans ladite utilisation, ledit polypeptide est utilisé avec un adjuvant stimulant la réponse immunitaire lorsqu'il est administré à un hôte humain ou animal.

6. Polypeptide selon l'une quelconque des revendications précédentes, pour son utilisation dans un vaccin selon la revendication 1 **caractérisé en ce que**, dans ladite utilisation, ledit polypeptide est utilisé avec du CpG.

7. Polypeptide selon l'une quelconque des revendications précédentes, pour son utilisation dans un vaccin selon la revendication 1 **caractérisé en ce que** ledit vaccin prévient ou traite une infection par *Leishmania major.*

8. Molécule d'acide nucléique codant un polypeptide tel que défini à l'une quelconque des revendications 1 à 7, pour son utilisation dans un vaccin permettant d'obtenir une protection contre une infection par *Leishmania.*

9. Molécule selon la revendication 8, pour son utilisation dans un vaccin selon la revendication 8 **caractérisée en ce que**, dans ladite utilisation, ladite molécule est utilisée à titre d'agent induisant ou favorisant l'activation de cellules effectrices de l'immunité à médiation cellulaire.

10. Molécule selon l'une quelconque des revendications 8 à 9, pour son utilisation dans un vaccin selon la revendication 8 **caractérisée en ce que**, dans ladite utilisation, ladite molécule est utilisée à titre d'agent induisant ou favorisant une réponse immunitaire de type Th1.

11. Molécule selon l'une quelconque des revendications 8 à 10, pour son utilisation dans un vaccin selon la revendication 8 **caractérisée en ce que**, dans ladite utilisation, ladite molécule est utilisée avec un adjuvant stimulant la réponse immunitaire lorsqu'il est administré à un hôte humain ou animal.

12. Molécule selon l'une quelconque des revendications 8 à 11, pour son utilisation dans un vaccin selon la revendication 8 **caractérisée en ce que**, dans ladite utilisation, ladite molécule est utilisée avec du CpG.

13. Molécule selon l'une quelconque des revendications 8 à 12, pour son utilisation dans un vaccin selon la revendication 8 **caractérisée en ce que** ledit vaccin prévient ou traite une infection par *Leishmania major.*

14. Molécule selon l'une quelconque des revendications 8 à 13, pour son utilisation dans un vaccin selon la revendication 8 **caractérisée en ce qu'**elle est la molécule d'acide nucléique codante qui est portée par le plasmide pET-NH2B déposé auprès la Collection Nationale de Cultures de Micro-organismes (CNCM) sous le n° I-3363.

## Patentansprüche

1. Polypeptid bestehend aus einem N-terminalen Fragment des Histon-Proteins H2B von *Leishmania* zur Verwendung in einem Impfstoff, der Schutz gegen eine Infektion durch Leishmania ermöglicht, wobei die Aminosäuresequenz des N-terminalen Fragments eine Sequenz ist, kodiert durch:

2. Polypeptid nach Anspruch 1 zur Verwendung in einem Impfstoff nach Anspruch 1, dessen Aminosäuresequenz
MASSRSASRKASNPHKSHRKPKRSWNVYVGRSLKAINAQMSMSHRT ist.

3. Polypeptid nach einer beliebigen der vorhergehenden Ansprüche, zur Verwendung in einem Impfstoff nach Anspruch 1, **dadurch gekennzeichnet, dass** das Polypeptid in der Verwendung als Mittel verwendet wird, das die Aktivierung von Effektorzellen der zellvermittelten Immunität induziert oder fördern.

4. Polypeptid nach einem beliebigen der vorhergehenden Ansprüche zur Verwendung als Impfstoff nach Anspruch 1, **dadurch gekennzeichnet, dass** das Polypeptid in der Verwendung als Mittel verwendet wird, das eine Immunantwort des Typs Th1 induziert oder fördert.

5. Polypeptid nach einem beliebigen der vorhergehenden Ansprüche zur Verwendung in einem Impfstoff nach Anspruch 1, **dadurch gekennzeichnet, dass** das Polypeptid in der Verwendung mit einem Adjuvans verwendet wird, das, wenn es einem menschlichen oder tierischen Wirt verabreicht wird, die Immunantwort stimuliert.

6. Polypeptid nach einem beliebigen der vorhergehenden Ansprüche zur Verwendung in einem Impfstoff nach Anspruch 1, **dadurch gekennzeichnet, dass** das Polypeptid in der Verwendung mit CpG verwendet wird.

7. Polypeptid nach einem beliebigen der vorhergehenden Ansprüche zur Verwendung in einem Impfstoff nach Anspruch 1, **dadurch gekennzeichnet, dass** der Impfstoff einer Infektion durch *Leishmania major* vorbeugt oder diese behandelt.

8. Nukleinsäuremolekül kodierend ein Polypeptid wie in einem beliebigen der Ansprüche 1 bis 7 definiert, zur Verwendung in einem Impfstoff, der Schutz gegen eine Infektion durch *Leishmania* ermöglicht.

9. Molekül nach Anspruch 8, zur Verwendung in einem Impfstoff nach Anspruch 8, **dadurch gekennzeichnet, dass** das Molekül in der Verwendung als Mittel verwendet wird, das die Aktivierung von Effektorzellen der zellvermittelten Immunität induziert oder fördert.

10. Molekül nach einem beliebigen der vorhergehenden Ansprüche 8 oder 9 zur Verwendung in einem Impfstoff nach Anspruch 8, **dadurch gekennzeichnet, dass** das Molekül in der Verwendung als Mittel verwendet wird, das eine Immunantwort des Typs Th1 induziert oder fördert.

11. Molekül nach einem beliebigen der vorhergehenden Ansprüche 8 bis 10 zur Verwendung in einem Impfstoff nach Anspruch 8, **dadurch gekennzeichnet, dass** das Molekül in der Verwendung mit einem Adjuvans verwendet wird, das, wenn es einem menschlichen oder tierischen Wirt verabreicht wird, die Immunantwort stimuliert.

12. Molekül nach einem beliebigen der vorhergehenden Ansprüche 8 bis 11 zur Verwendung in einem Impfstoff nach Anspruch 8, **dadurch gekennzeichnet, dass** das Molekül in der Verwendung mit CpG verwendet wird.

13. Molekül nach einem beliebigen der vorhergehenden Ansprüche 8 bis 12 zur Verwendung in einem Impfstoff nach Anspruch 8, **dadurch gekennzeichnet, dass** der Impfstoff einer Infektion durch *Leishmania major* vorbeugt oder diese behandelt.

14. Molekül nach einem beliebigen der vorhergehenden Ansprüche 8 bis 13 zur Verwendung in einem Impfstoff nach Anspruch 8, **dadurch gekennzeichnet, dass** es das kodierende Nukleinsäuremolekül ist, das von dem Plasmid pET-NH2B getragen wird, hinterlegt unter der Nr. I-3363 bei der Collection Nationale de Culture de Microorganismes (CNCM).

## Claims

1. A polypeptide consisting of an N-terminal fragment of the H2B histone protein of *Leishmania,* for use thereof in a vaccine for obtaining protection against an infection by *Leishmania,* the amino acid sequence for said N-terminal fragment being a sequence encoded by:

2. The polypeptide according to claim 1 for use thereof in a vaccine according to claim 1, wherein the amino acid sequence is:
MASSRSASRKASNPHKSHRKPKRSWNVYVGRSLKAINAQMSMSHRT.

3. The polypeptide according to any one of the preceding claims for use thereof in a vaccine according to claim 1, **characterized in that** in said use, said polypeptide is used as an agent inducing or promoting the activation of cell-mediated immunity effector cells.

4. The polypeptide according to any one of the preceding claims for use thereof in a vaccine according to claim 1, **characterized in that** in said use, said polypeptide is used as an agent inducing or promoting a type Th1 immune response.

5. The polypeptide according to any one of the preceding claims for use thereof in a vaccine according to claim 1, **characterized in that** in said use, said polypeptide is used with an adjuvant stimulating the immune response when it is administered to a human or animal host.

6. The polypeptide according to any one of the preceding claims for use thereof in a vaccine according to claim 1, **characterized in that** in said use, said polypeptide is used with CpG.

7. The polypeptide according to any one of the preceding claims for use thereof in a vaccine according to claim 1, **characterized in that** said vaccine prevents or treats an infection by *Leishmania major.*

8. A nucleic acid molecule encoding a polypeptide as defined in any one of claims 1 to 7, for use thereof in a vaccine for obtaining protection against an infection by *Leishmania.*

9. The molecule according to claim 8 for use thereof in a vaccine according to claim 8, **characterized in that** in said use, said molecule is used as an agent inducing or promoting the activation of cell-mediated immunity effector cells.

10. The molecule according to claim 8 or claim 9, for use thereof in a vaccine according to claim 8, **characterized in that** in said use, said molecule is used as an agent inducing or promoting a type Th1 immune response.

11. The molecule according to any one of claims 8 to 10, for use thereof in a vaccine according to claim 8, **characterized in that** in said use, said molecule is used with an adjuvant stimulating the immune response when it is administered to a human or animal host.

12. The molecule according to any one of claims 8 to 11, for use thereof in a vaccine according to claim 8, **characterized in that** in said use, said molecule is used with CpG.

13. The molecule according to any one of claims 8 to 12, for use thereof in a vaccine according to claim 8, **characterized in that** said vaccine prevents or treats an infection by *Leishmania major.*

14. The molecule according to any one of claims 8 to 13, for use thereof in a vaccine according to claim 8, **characterized in that** it is the encoding nucleic acid molecule that is carried by the plasmid pET-NH2B deposited with the National Collection of Microorganism Cultures (CNCM) with accession number I-3363.
